# EUROPEAN PATENT APPLICATION

(11) **EP 0 538 895 A2**
(43) Date of publication of application: **28.04.1993**
(21) Application number: 92118217.6
(22) Date of filing: 23.10.1992
(51) Int. Cl.: A61B 17/58, A61F 2/36, A61F 2/38, A61B 17/064

(54) **Cement-free endosprosthesis**

(30) Priority: 25.10.1991 DE 4135310
(71) Applicant: Habermeyer, Peter, Dr., D-81925 München (DE)
(72) Inventor: Habermeyer, Peter Dr., W-8000 München 81 (DE); Tornier, Alain, F-38300 Saint Ismier (FR)
(74) Representative: Dipl.-Phys.Dr. Manitz Dipl.-Ing. Finsterwald Dipl.-Ing. Grämkow Dipl.Chem.Dr. Heyn Dipl.Phys. Rotermund Morgan, B.Sc.(Phys.)

(57) **Abstract**

The invention relates to a cement-free endoprosthesis with an anchoring part (1) having a thread which is to be screwed into a bone (3) and also a joint surface replacement part (2). In this endoprosthesis the anchoring part (1) has a hollow cylindrical region (10) which is provided with a thread and which has a free axially open end which on being screwed in, penetrates into the bone (3) while forming a circular cutting path. The anchoring part (1) is provided at its end face remote from the open end of the hollow cylindrical region (10) with a head region (14) which can be coupled to the joint surface replacement part (2).

## Description

The invention relates to a cement-free endoprosthesis with an anchoring part which is to be screwed into a bone and which has a thread and also a joint surface replacement part.

An endoprosthesis of this kind is for example known from European patent application, publication no. 0 274 094. For the insertion of this prothesis a relatively large space must be reamed out in the bone in order to provide space for the prothesis which is to be inserted which is secured in the opening provided in the bone by means of a conical screw-like threaded part the thread surfaces of which project out of side windows of the endoprosthesis.

Other known endoprostheses are provided with anchoring projections which are cemented into hollowed out openings of the bone. These endoprostheses are frequently additionally screwed to the bone after they have been cemented in.

It is a disadvantage of these known prostheses that the endoprostheses which are to be cemented in can become loose in the course of time and that in all such endoprostheses the bone can be weakened by the hollowing out so that the loadability is impaired.

The object of the present invention is to provide a permanently durable endoprosthesis which can be used without prior hollowing out of the bone.

The object of the invention is satisfied by an endoprosthesis in accordance with the invention in that the anchoring part has a hollow cylindrical region which is provided with the thread and has a free axially open end which penetrates into the bone on being screwed in while forming a circular cutting path in the bone, and in that the anchoring part is provided at its end face remote from the open end of the hollow cylindrical region with a head region which can be coupled to the joint surface replacement part.

Through the design of the anchoring part with a hollow cylindrical region which is provided with the thread, the anchoring part can be screwed into the bone without the bone having to be previously hollowed out. The hollow cylindrical region of the anchoring part thereby penetrates into the spongy region of the bone and there the thread finds a hold in the bone. The hollow cylindrical shape ensures a large area anchorage. The modularly constructed endoprosthesis comprising an anchoring part and joint surface replacement part is simple to position and to permanently fix in the bone. The endoprosthesis of the invention is particularly suitable for use as an unblocked prothesis.

A particularly preferred embodiment of the invention is characterised in that the said anchoring part comprises first and second parts, namely a generally planar base plate for mounting on a cut and optionally prepared end surface of a bone and a hollow screw insertable into said bone through an aperture provided in said base plate. The hollow screw can conveniently have a flange at a head end thereof which engages over at least a portion of said base plate surrounding said aperture. Further expedient and useful developments of this embodiment are set forth in the claims 11 to 29.

The thread is preferably formed at the outer periphery of the outer cylindrical region whereby the anchoring takes place over a maximum area. A sharp edged circular ring-like end face of the hollow cylindrical region at the open end permits simple placement and penetration of the hollow cylindrical region into the bone.

If the preferably shallow and planar head region is formed for the engagement of a tool then the anchoring part can be particularly easily screwed into the bone.

A design in which the head region of the prosthesis is formed for the rotationally fixed and axially stable positioning of the joint surface replacement part which is mountable thereon permits a simple and rotationally secure positioning of the joint surface replacement part. If the head region is made multicornered at its peripheral edge, and if the joint surface replacement part is provided with a matching multicornered mount for the head region then the joint surface replacement part can be definitely and reliably mounted on the anchoring part in different angular positions and can thus be adapted to the geometrical and kinematic requirements in the region of the joint.

An alternative embodiment of the invention which achieves the same advantages as the previously described embodiment, namely a mounting of the anchoring part without the anchoring part having to be cemented in, or without the bone having to be hollowed out, is characterized in that the anchoring part is of cramp-like form, with the two free limbs being provided with lateral hooking projections which on hammering in or shooting in of the anchoring part into the bone become hooked in the bone; and in that the web of the anchoring part connecting the free limbs can be coupled to the joint surface replacement part.

Further advantageous forms of the invention are also set forth in the remaining claims.

The invention will subsequently be explained in more detail with reference to examples and to the drawings in which are shown:
- Figure 1: a sectioned spatial view of a cement-free endoprosthesis of the invention,
- Figure 2: a partly transparent plan view onto an endoprosthesis in accordance with the invention,
- Figure 3: a schematic sectional view of an anchoring part inserted into a bone,
- Figure 4: a schematic sectional view of an endoprosthesis in accordance with the invention inserted into a bone,
- Figure 5: the use of an endoprosthesis as a hip-joint head prothesis with an arthrotic change of the hip-joint head,
- Figure 6: the representation of a damaged joint surface of an ankle joint,
- Figure 7: the same ankle joint with an endoprosthesis in accordance with the invention inserted in place of the damaged joint surface,
- Figure 8: the use of an endoprosthesis in the knee joint,
- Figure 9: an alternative endoprosthesis in accordance with claim 30 of the invention,
- Figure 10: a plan view of a base plate of an alternative design of anchoring part,
- Figure 11: a side view of the base plate of Figure 10 as seen in the direction of the arrow XI,
- Figure 12: a view of the rear side of the base plate of Figure 10,
- Figure 13: a side view of a half turn locking screw for use with the base plate of Figure 10,
- Figure 14: a view of the top of the half turn locking screw of Figure 13 as seen in the direction of the arrow XIV,
- Figure 15: a side view of a hollow screw for use with the base part and locking screw of Figs. 10 to 14 but with a simpified representation of the thread at the cylindrical wall of the hollow screw,
- Figure 16: a plan view onto the head of the hollow screw of Figure 15 as seen from above in the direction of the arrow XVI,
- Figure 17: a detail of the thread provided on the cylindrical wall of the hollow screw,
- Figure 18: a perspective view of a first cap member for use with the base plate and hollow screw of the embodiment of Figures 10 to 17,
- Figure 19: a plan view of the top of the cap member of Figure 18 as seen in the direction of the arrow XIX,
- Figure 20: a side view of the cap member of Figure 18 as seen in the direction of the arrow XX,
- Figure 21: a simplified, schematic partial cross-section through the cap member of Figure 18 when installed on a base plate in accordance with Figures 10 to 12 representing a cross-section taken on the plane XXI-XXI of Fig. 19,
- Figure 22: an end view of the cap member of Figure 19 in the direction of arrow XXII showing a disengaging slot,
- Figure 23: a plan view similar to that of Figure 19 but of a modified embodiment.

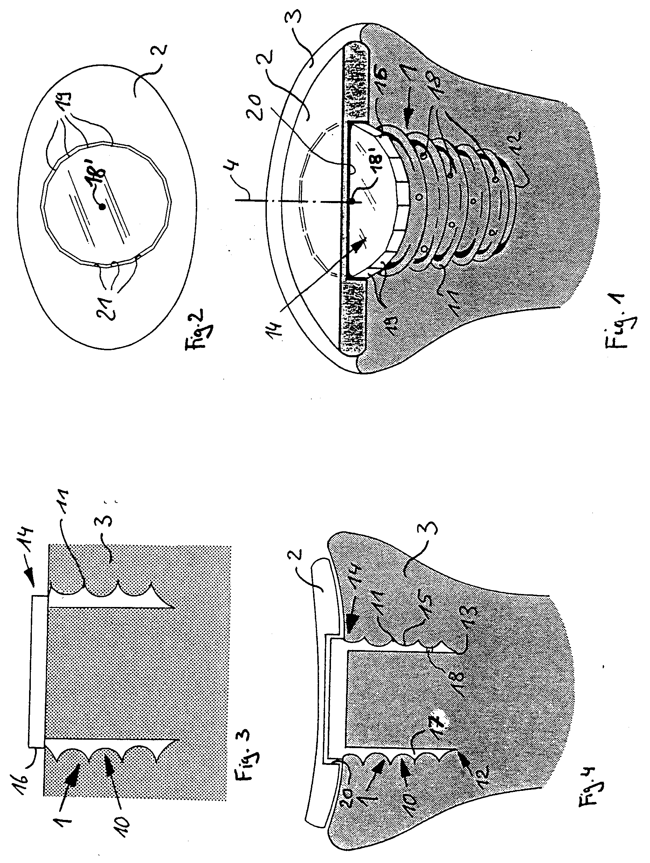

In Figure 1 there is shown the joint region of a bone 3 in which an endoprosthesis in accordance with the invention is inserted. The hollow cylindrical region 10 (Figure 4) of the anchoring part 1 which can consist of several hollow cylindrical sections separated from one another, is provided at its outer periphery 15 with a thread and is axially inserted essentially centrally into the bone while forming a cylindrical cutting path.

In this region the bone is, on the one hand, spongy, so that the screwing in of the anchoring part can take place without excessive expenditure of force, is however, on the other hand, sufficiently firm that a permanent and stable fixing of the anchoring part is possible. Additional screw fastenings in the harder edge zones of the bone are not necessary when inserting this endoprosthesis.

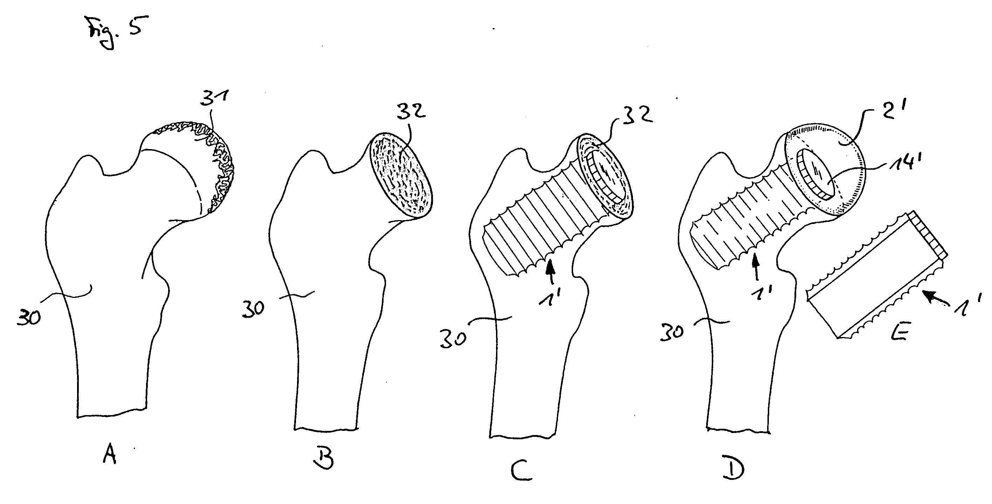

The anchoring part 1 is provided at its end face remote from the open end 12 in the bone with a head region 14. The head region 14 is preferably shaped so that it is shallow and flat or planar as can be seen in Figures 3 and 4. At its peripheral edge 16 the head region 14 is of many cornered form and thus has a plurality of flat peripheral surfaces 19 distributed around the periphery essentially at right angles to the upper planar surface of the head region 14. In the embodiment shown in Figures 1 and 2 sixteen surfaces 19 are provided over the periphery.

A joint surface replacement part 2 is coupled to the head part 14 of the anchoring part 1 and has a central recessed mount 20 having at its inner periphery the same number of essentially vertically (in Figure 1) extending inner peripheral surfaces 21. In this manner the joint surface replacement part 2 can be inserted into a plurality of positions displaced around the axis 4 of the anchoring part 1, or of the joint surface replacement part 2, relative to the anchoring part before it is secured to the bone or to the anchoring part.

Openings 18 are provided in the wall 17 of the cylindrical region 10 of the anchoring part 1. These serve to permit the bone to grow into them and hereby ensure additional anchoring stability. In the head region 14 there is provided at least one axially directed opening 18' which, on the one hand, serves for the emergence of air and liquid and on the other hand as an installation aid .

The installation of the anchoring part takes place in such a way that first of all a drill target wire is inserted into the bone and aligned. The anchoring part is then so mounted that the drill target wire passes through the central opening 18' in the head region 14 of the anchoring part 1. The anchoring part is then screwed into the bone guided by the drill target wire.

In Figure 5 there is shown the use of the endoprosthesis of the invention in a hip-joint. In the part drawing A there is shown a thigh bone femur with a damaged joint surface 31. The partial illustration B shows the thigh bone femur 30 in which the arthrotic femur head 31 has been removed. The anchoring part 1' of the endoprosthesis of the invention is screwed into the cut surface 32 which has arisen through the removal of the femur head (partial illustration C). In partial illustration D there can be seen the finished state in which the joint replacement part 2'' is coupled to the head part 14' of the anchoring part 1'. The partial illustration E shows the extra long anchoring part 1' used in this endoprosthesis, which ensures a reliable support for the forces and moments which are to be introduced via the joint surface replacement part 2' into the bone.

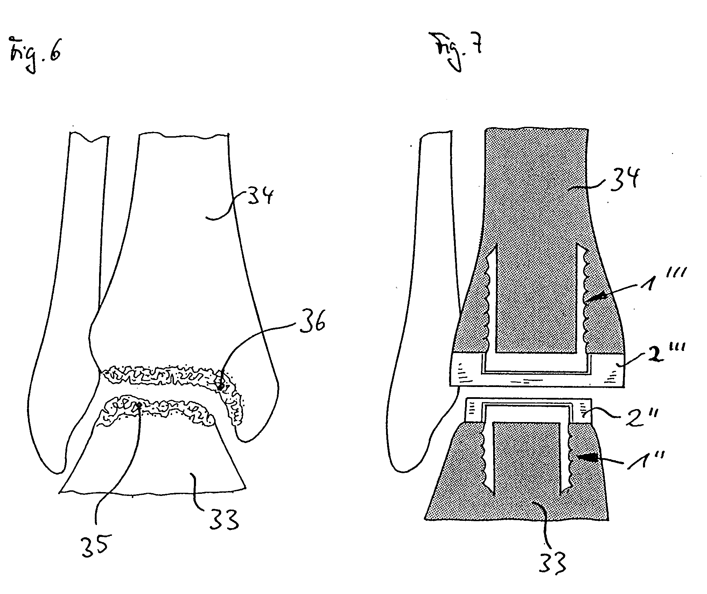

In Figure 6 there is shown an upper ankle joint with damaged joint surfaces 35, 36. Both the joint surface 35 of the ankle bone and also the joint surface 16 of the tibia 34 are replaced by an endoprosthesis in accordance with the invention. This use as an unblocked joint in which the lower and the upper endoprosthesis are held together only by muscle power and without the use of connective artificial elements is shown by Figure 7. In this arrangement an anchoring part 1'' is inserted into the ankle bone 33 in place of the removed joint surface 35 and the joint surface replacement part 2'' is attached to the anchoring part 1''. At the opposite side an anchoring part 1'' is inserted into the tibia 34 in place of the removed joint surface 36 of the tibia 34 and a joint surface replacement part 2''' is mounted onto the anchoring part 1'''. The two oppositely disposed joint surface displacement parts 2'' and 2''' cooperate in the same way as the natural joint surfaces 35 and 36. The endoprosthesis in accordance with the invention is moreover particularly suited for shoulder and hand joints and also for use in the knee joint and also in the dentistry.

Figure 8 shows the use of an endoprosthesis in accordance of the invention in a knee joint. Here the anchoring part 101'' which is provided with a lower joint surface replacement part 102'' is inserted into the tibia 34 while the anchoring part 101''' connected to the upper joint surface 102''' is inserted into the thigh bone 37.

Figure 9 shows an alternative embodiment in which a cramp-like anchoring part 201 is fixed with its two free limbs 210, 211, which are provided with outwardly projecting hook formations 212, into the bone 203 for example by being hammered in or by being shot in. The bridge region 213 which connects the two free limbs 210, 211 is formed in the manner known from the first variant for coupling with the joint surface replacement part.

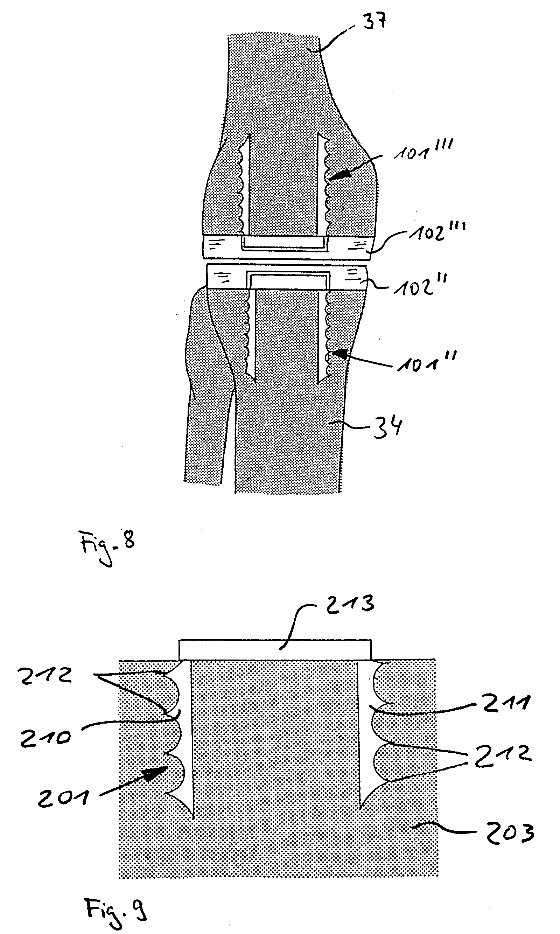

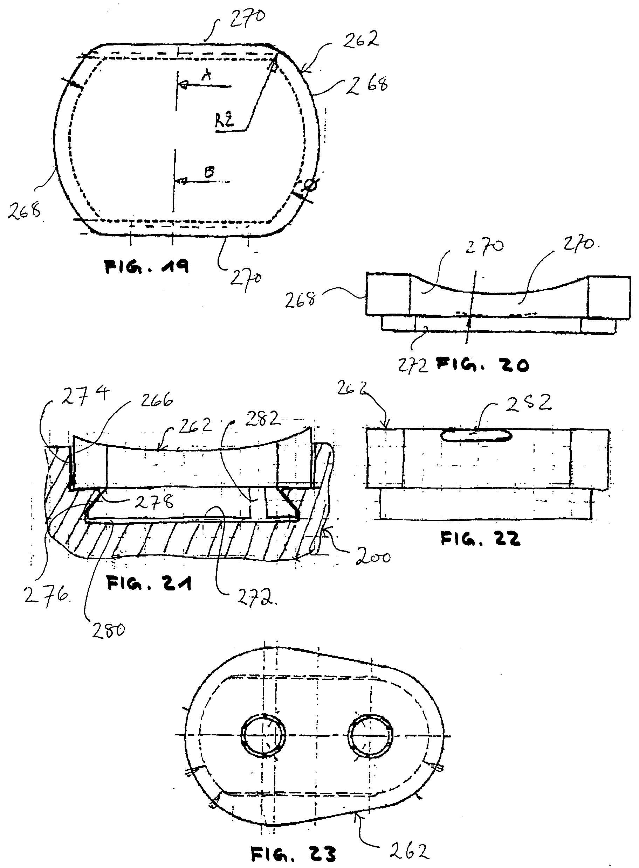

In a preferred embodiment of the invention an anchoring part 1 is formed of two major parts, namely a base plate 200 shown in Figures 10, 11 and 12 and a hollow screw 202 shown in Figures 15, 16 and 17. The anchoring part shown in these figures is primarily intended for use as a cement-less glenoid prosthesis system and would normally be mounted on the cut surface of the shoulder bone which receives the head of the humerus, i.e. to a cut surface similar to the example 32 given for the femur head in Figure 5B. Although the cement-free endoprosthesis described in the following with respect to Figures 10 to 23 specifically relates to the replacement of the shoulder joint cup it will be clearly understood that a similar design could also be used for other joint replacements with adaptation to the particular shape and size of the joint/bone involved. The rear surface 204 of the base plate 200 is mounted in use against the cut surface. The base plate is secured in place on the cut surface by the hollow screw 202 which passes through the central aperture 206 of the base plate from the front side 208 shown in Figure 10 and which is screwed on until the head flange 210 of the hollow screw sits against the ring seat 212 surrounding the aperture 206 on the base plate.

In practice the aperture 206 in the base plate of Figure 10 can be used as a guide for the insertion of the hollow screw 202 after it has been accurately positioned on the cut glenoid surface.

It will noted from Fig. 11 that the base plate 200 has two tapering hollow projections or spigots 214 and 216 projecting from the rear side of the base plate, with the spigots being provided on opposite sides of the aperture 206 on a straight line passing through the center 218 of the aperture. The purpose of these tubular spigots 214, 216 is to provide an anti-rotational device which prevents rotation of the base plate relative to the cut glenoid surface.

After the formation of the cut glenoid surface, and possibly dressing of the surface to ensure that it is flat, it will be prepared for the application of the base plate. A template will typically be fitted over the glenoid surface and is used to drill two holes into the glenoid substantially perpendicular to its surface, at positions correspoonding to the positions of the two spigots 214 and 216. The holes would provide a light interference fit for the tapering spigots 214 and 216 so that after removal of the template the base plate 216 can be tapped into place on the end of the cut glenoid surface. It will be noted that the rear surface 204 of the base plate 200 is provided with diamond shaped pyramids 220 as indicated by the cross-hatching in Fig. 12 rather like a minituarised steak tenderising hammer. The surface features in the form of the diamond-shaped pyramids 220, can also be of other shapes, and are intended to help the bone to grow around the prosthesis following fitting of the same.

After the base plate has been fitted to the cut glenoid surface by tapping the tapering spigots 214 and 216 into place the hollow screw 202 is placed in the aperture 206 and rotated by means of a tool placed into the torque application recess 224 of the essentially closed end face of the hollow screw. In fact the base of the tool recess 224, which can for example be a hexagonal or other type screwing hole, as conveniently left open but could be closed if desired.

It can be seen from Fig. 16 that the hollow screw 202 in fact has a serrated collar 225 at its head flange 210 with a plurality of part circular serrations 226 each of which has an angled side surface 228.

Moreover, it will be noted that the hollow screw 202 has a ring shoulder 230 immediately beneath the head flange 210 which can form a good fit with the inner edge of the aperture 206 of the base plate 200 to ensure reliable transverse location on the base plate on the cut glenoid surface. In addition, it will be noted from Fig. 15 that the hollow screw has a plurality of apertures 232 in its side wall which promote bone growth through the wall of the hollow screw and improving the anchoring of the same. The screw thread 234 on the side wall of the hollow cylindrical portion 236 of the hollow screw is shown in detail in Fig. 17. It will be noted that the thread has a first flank 238 which extends substantially perpendicular to the central axis 240 of the hollow screw and a second flank 242 at an acute angle to the first flank 238. The flank 238 perpendicular to the axis 240 of the screw (at least when viewed at any longitudinal section through the screw including the central axis 240) means that pressure loads acting on the bone as a result of the prosthesis will be born largely as compressive loads without any tendency to expand the bone as would be the case if the flange 238 were an angled flange similar to the flange 242.

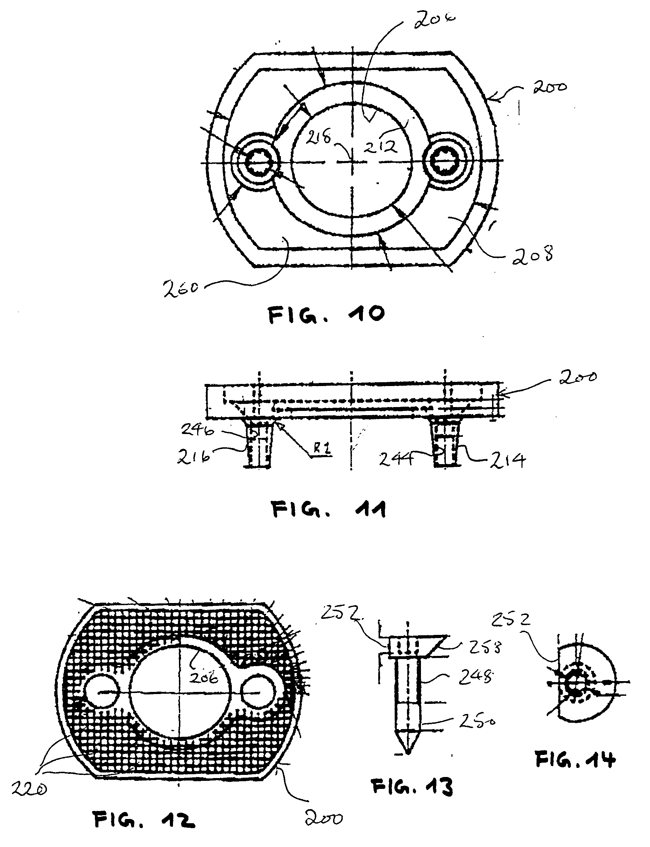

Figs. 13 and 14 show two views of a half turn locking screw such as is inserted into each of the tubular spigots 214, 216 from the top side 208 of the base plate 200. For this purpose the hollow tubular spigots 214, 216 are provided with an internal thread 244 and 246 respectively into which a corresponding threaded portion 248 of the respective locking screws engages. This means that the point portion 250 of the locking screw projects beneath the bottom of the two hollow spigots 214, 246 in use.

The threaded engagement between the locking screws and the tubular spigots 214, 216 serves two primary purposes. First of all it permits the locking screws to be mounted in the base plate 200 so that they cannot easily be lost and are in fact fitted together with the base plate onto the cut glenoid surface.

Secondly the locking screws serve to lock the hollow screw into position once this has been fitted. For this purpose the flat side 252 of the essentially D-shaped head of each locking screw is initially rotated so that it does not interfere with rotation of the head flange 210 of the hollow screw on fitting of the hollow screw 202. The thin-walled spigots 214 and 246 can, if desired, be slightly deformed from the round shape so that there is a slight amount of friction which prevents unwanted rotation of the locking screws. Alternatively the tip end 250 of the locking screws engaging the bone can be a sufficiently tight-fit in the bone that such undesired rotation is prevented. Once the hollow screw has been fitted it is tightened so that a pair of oppositely disposed serrations 226 lie with their centers of curvature on the diameter 256 and the two locking screws are then rotated so that the circular tapered flank 258 of the head of the locking screw comes into engagement with the correspondingly tapered flank 228 of the relevant serration. Thereafter the locking screws prevent undesired rotation of the hollow screw.

It will be noted that the locking screws could for example also be executed as pins which are tapped into place after fitting of the hollow screw to prevent rotation of the same.

The cement-less glenoid prosthesis system is then completed by a cap member 262, which is expediently of a clinically approved plastic material which is fitted into a recess 260 of the top surface of the base plate (which can best be seen from Fig. 21).

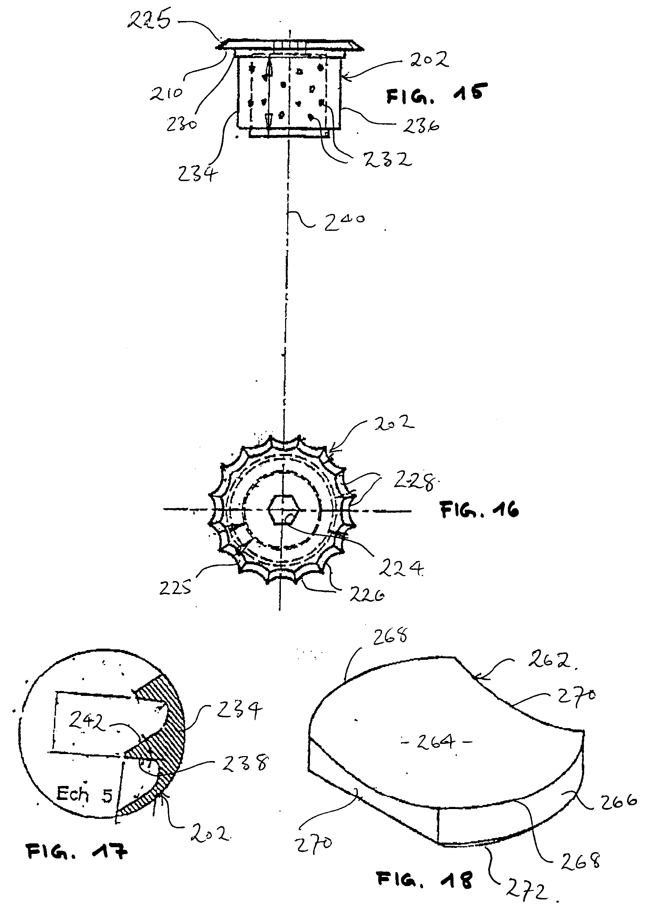

The cap member 262 is shown detail in Figures 18 to 22. As can be seen from the perspective view of Figure 18 it has a top part spherically shaped surface 264 which in plan view resembles a flattened circle as can be readily seen from Figure 19. In fact this shape is often described as "Neer-shape" having regard to the first development of the replacement joints of this external profile by a surgeon called "Neer". As can be seen from Figure 18 and also from Figure 20 the cap member 262 is of a fairly shallow shape, so that there is no change in the geometry of the original joint. The cap member has therefore only a shallow side surface 266 which can be thought of as two confronting circular arcs 268 joined by two flat side edges 270. Projecting from the underside of the cap member is short recessed portion 272 which can best be seen from Figure 20 and from Figure 21. It will be noted that the outline of the projection 272 (which can be seen from Figure 19) corresponds in shape to that of the outer contour of the cap member 262 but is actually of a smaller size so that it can fit into the recess 208 of the base member. Moreover, as can be seen from Fig. 21 the recess in the base member has first side 274 which cooperates with the side wall 266 of the cap member and disposed within this an undercut side surface 276 which cooperates with a complementary shaped inclined side surface 278 of the projection 272. As can be seen from Fig. 21 the projection 272 and the recess 280 defined by the inclined side walls 276 of the base part 202 of the insert together form a dovetail fitting which retains the cap member 262 in the base plate. To permit the cap member to be snapped into place there is provided a slot 282 at one side of the projection 272 which permits the doughtail portion 284 at the right hand side in the view of Figure 21 to be deflected inwardly, thus partially closing the cap 282 so that the cap member can be snapped into place in the base member. Moreover, the cap member 262 is conveniently provided with a disengaging slot 286 through which for example a screwdriver can be inserted and pressed against the base plate at the top of hollow screw in order to remove the cap member, should this prove necessary. The plastic cap member or insert is first inserted into the bone plate on one side and then the flexible bit of the insert is pressed against the groove of the metal plate in order to obtain some space. The insert can then be snapped on. The other side of the dovetail of the insert engages into a groove of the plate and the flexible end flips back into this groove. The system is locked, it can be separated by means of a screwdriver and the slot in the plastic.

Finally, Fig. 23 shows an alternative pear-like contour of the cap member.

Thus the invention of Figs. 10 to 23 provides a cement-less glenoid prosthesis system in a modular form with three components, namely the metal back or base plate 200 sitting flat on the resurfaced glenoid surface and accepting the following two items:
- a polyethelene (or other material) insert for the reconstruction of the articulating surface of the gleno-humeral joint, and
- a hollow screw for firm and secure anchorage into the bone of the glenoid beneath the metal back surface.

1. The metal back is the shell of the system. It is first put in place in a centered position after resurfacing of the glenoidal bone, it serves as a guide for the machining of the bone to accept the screw guided through it and locked onto it. Alternately the trial plate or template can be used for this purpose. The metal back has antirotational attachments underneath. Its height is minimal to ensure small overall height for the system but maximum plastic height for the insert and optimum stability.
   Special surface aspects can be machined on or applied to its surface in contact with the bone for stability and bone ingrowth.
   The screw is the anchoring device on the bone and is designed and built to preserve a maximum of good bone stock where it matters and also to promote bone regrowth through it by preserving vascularisation and thanks to small holes in the walls of threaded cylinder which has a large hollow inside diameter and thin walls. Primary mechanical stability and secondary biological stability are then obtained. Its collar, serrated, allows it to be screwed completely in position while still being able to lock it by means of small screws in the metal plate.
   The insert is the "kinematic" element of the system, the female counterpart of the humeral head. It is snapped onto the metal back plate once the hollow screw is in place.
2. The system enables the following variations to optimise the fit to the anatomy:
   - a pear-like or Neer-like or any other shape of contour for the glenoid component,
   - different contour size for the glenoid (but the same height),
   - various sphere radius (or radii) in the case of a pear-like shape on the insert articulating surface facing the humeral head,
   - various cement-less surface aspects beneath the plate,
   - various heights, diameters and wall thicknesses of the hollow screw,
   - various surface aspects can also be accommodated,
   - various antirotational details underneath the plateau for stability, spikes, tapered pins with or without throughscrews.
3. The system is such that it allows any combination of the variations mentioned above under heading 2) to be used simultaneously.
4. The system is such that any material can be used if clinically acceptable.
5. The system is such that the assembly and locking of the hollow screw on the metal back plate is gap-free and provides additional stability of the system onto the bone. Specially designed ancillary equipment helps in that.
6. The system is such that the locking of the hollow screw on the plate is achieved by securing it in position with two "half turn" screws engaging in the serrations of the collar of the hollow screw. Numerous such serrations allow this to take place whichever angular position is taken by the hollow screw when it bottoms on the metal back surface.
   The screws on the metal plate are preferentially captive.

## Claims

1. Cement-free endoprosthesis comprising an anchoring part having a thread to be screwed into a bone and also a joint surface replacement part,
characterised in that
the anchoring part (1) has a hollow cylindrical region (10) which is provided with the thread (11) and has a free axially open end (12) which penetrates into the bone (3) on being screwed in while forming a circular cutting path in the bone (3), and in that the anchoring part (1) is provided at its end face remote from the open end (12) of the hollow cylindrical region (10) with a head region (14) which can be coupled to the joint surface replacement part (2).

2. Cement-free endoprosthesis in accordance with claim 1,
characterised in that
the thread (11) is formed at the outer periphery (15) of the hollow cylindrical region (10).

3. Cement-free endoprosthesis in accordance with claim 2,
characterised in that
a sharp edged circular ring-like end face (13) is formed at the open end (12) of the hollow cylindrical region.

4. Cement-free endoprosthesis in accordance with claim 1,
characterised in that
the preferably shallow and planar head region (14) is formed for the engagement of a tool.

5. Cement-free endoprosthesis in accordance with claim 1,
characterised in that
the head region (14) is formed for rotationally fixed and axially stable positioning of the joint surface replacement part (2) mountable thereon.

6. Cement-free endoprosthesis in accordance with claim 4,
characterised in that
the head region (14) is made multi-cornered at its peripheral edge (16) and in that the joint surface replacement part (2) has a matching multi-cornered receiving mount (20) for the head region (14).

7. Cement-free endoprosthesis in accordance with claim 1,
characterised in that
the head region (14) forms a substantially closed end face of the anchoring part (1).

8. Cement-free endoprosthesis in accordance with claim 1,
characterised in that
openings (18) are present in a wall (17) of the hollow cylindrical region (10) and/or in the end face (13) of the head region (14).

9. Cement-free endoprosthesis in accordance with claim 1,
characterised in that
said anchoring part comprises first and second parts namely a generally planar base plate for mounting on a cut and optionally prepared end surface of a bone and a hollow screw insertable into said bone through an aperture provided in said base plate.

10. Cement-free endoprosthesis in accordance with claim 9,
characterised in that
said hollow screw has a flange at a head end thereof which engages over at least a portion of said base plate surrounding said aperture.

11. Cement-free endoprosthesis in accordance with claim 9,
characterised in that
said base plate has an outer boundary which is generally pear shaped.

12. Cement-free endoprosthesis in accordance with claim 9,
characterised in that
said base plate has an outer boundary which substantially comprises two approximately semi-circular arcs connected by two preferably straight lines.

13. Cement-free endoprosthesis in accordance with claim 12,
characterised in that
said two circular arcs are of the same radius of curvature thus resulting in a shape for said base plate representing a Neer-shaped glenoid endoprosthesis.

14. Cement-free endoprosthesis in accordance with claim 12,
characterised in that
said circular arcs are of different diameters and/or peripheral lengths thus resulting in a pear shaped base plate.

15. Cement-free endoprosthesis in accordance with claim 9,
characterised in that said base plate includes means disposed to one side of a central axis of said aperture and engaging in said bone to locate the base plate thereon and to prevent rotational movement thereof relative to said bone.

16. Cement-free endoprosthesis in accordance with claim 15,
characterised in that said means comprises at least one hollow spigot provided on said base plate.

17. Cement-free endoprosthesis in accordance with claim 14,
characterised in that
said means comprises a pin, nail or screw insertable through said base plate into said bone.

18. Cement-free endoprosthesis in accordance with claim 16,
characterised in that
said means comprises a pin, nail or screw insertable through said hollow spigot.

19. Cement-free prosthesis in accordance with claim 18,
characterised in that
said means comprises a headed pin, screw or nail having a locking feature and in that said locking feature can cooperate with said hollow screw to lock the same to prevent rotation relative to said base plate.

20. Cement-free endoprosthesis in accordance with claim 19,
characterised in that
said means comprises a headed screw having a threaded portion cooperating with a thread in said spigot, said screw having a first position with said locking feature remote from said head of said hollow screw and a second position rotated through approximately 180° relative to said first position for engagement with said head of said hollow screw.

21. Cement-free endoprosthesis in accordance with claim 20,
characterised in that
said hollow screw has a hollow cylindrical region with a thread and a free axially open end which penetrates into said bone on screwing said hollow screw into said bone.

22. Cement-free endoprosthesis in accordance with claim 21,
characterised in that
said thread of said hollow screw is an external thread and preferably has one flank directed substantially perpendicular to a central axis of said hollow screw.

23. Cement-free endoprosthesis in accordance with claim 22,
characterised in that
said hollow screw has holes in said hollow generally cylindrical region to promote bone regrowth and/or ingrowth.

24. Cement-free endoprosthesis in accordance with claim 9,
characterised in that
said joint replacement surface comprises a surface of a cap member insertable in or mountable on said base plate.

25. Cement-free endoprosthesis in accordance with claim 24,
characterised in that
said cap member can be snapped into position in a recess in said base plate and is of substantially the same outer peripheral shape.

26. Cement-free endoprosthesis in accordance with claim 25,
characterised in that
said cap and said base plate are connectable together by a snap fitting which in cross-section resembles a dovetail joint.

27. Cement-free prosthesis in accordance with claim 25,
characterised in that
means is provided on at least one of said cap member and said base plate for facilitating release of said cap member from said base plate.

28. Cement-free endoprosthesis in accordance with claim 19,
characterised in that said base plate has surface features at its side facing said end surface of said bone for promoting bone growth and/or anchorage of said base plate.

29. Cement-free prosthesis in accordance with claim 28,
characterised in that
said features comprise pyramid-shaped formations.

30. Cement-free endoprosthesis comprising an anchoring part having a thread which is to be screwed into a bone and also a joint surface replacement part,
characterised in that
the anchoring part (201) is of cramp-like form, with the two free limbs (210, 211) being provided with lateral hooking projections (212) which on hammering in or shooting in of the anchoring part into the bone become hooked in the bone and in that the web (213) of the anchoring part (201) connecting the free limbs can be coupled to the joint surface replacement part.
